# EUROPEAN PATENT SPECIFICATION

(11) **EP 1 333 768 B1**
(45) Date of publication and mention of the grant of the patent: **16.08.2006**
(21) Application number: 01978812.4
(22) Date of filing: 26.10.2001
(51) Int. Cl.: A61C 5/04, A61C 19/04

(54) **APPARATUS FOR TOOTH TREATMENT**
VorrichtUNG ZUR ZAHNBEHANDLUNG
APPAREIL DE TRAITEMENT DENTAIRE

(30) Priority: 26.10.2000 IS 568500
(43) Date of publication of application: 13.08.2003
(73) Proprietor: Globodent EHF., 600 Akureyri (IS)
(72) Inventor: JONSSON, Egill, 600 Akureyri (IS); GUDMUNDSSON, Geir, 107 Reykjavik (IS); VAN DER STRAATEN, Gerrit, 5062 ER Oisterwijk (NL); THORSTEINSSON, Sigurdur, I-20122 Milano (IT)
(74) Representative: Fridriksson, Einar Karl
(86) International application number: PCT/IS2001/000021
(87) International publication number: WO 2002/034154

(56) References cited:
- US-A- 5 545 039
- US-A- 5 562 448
- US-A- 5 567 156
- US-A- 5 697 787

## Description

### BACKGROUND AND PRIOR ART

Dental repair and dental and/or oral surgery involves precision and non-ergonomic work that creates much physical stress for dentists. A dentist's major working area is within the patient's mouth, which frequently puts the dentist in awkward positions, and hinders clear visibility of the full working environment. In order to have a better view of the patient's inner mouth, the dentist must often lean above the patient, and use mirrors to view "blind spots" known in the art as dead surfaces. Methods that alleviate some of the physical stress of dentists' work are therefore appreciated.

A significant portion of a dentist's work relates to the removal of dental material, such as of caries-affected tooth bone tissue or the shaping of tooth stumps for the insertion of dentures and artificial teeth. It is - in particular in the former case - of concern to the dentist not to remove more healthy bone tissue than necessary, as the outer parts of the teeth, the enamel layer and the intermediate dentin layer are not regenerated once destroyed.

Repair of dental decay such as caries may be broadly divided into direct and indirect repair methods. In direct repair methods the caries is removed and the cavity thus created is further cleaned and prepared. Thereafter a filling material, most often dental mercury amalgam or a plastic composite material is inserted into the prepared cavity. The filling, once hardened, is finished to smoothen out any rough edges and essentially create a surface similar in morphology to the original tooth.

Indirect repair methods on the other hand, involve the use of prepared fillings, e.g. from ceramic material (porcelain or glass) or gold, that are cast in a mold made after the cavity which has been prepared as described above. The filling, which typically takes a couple of days to manufacture, is fastened in place with dental adhesive cement. Indirect methods have been used for a long time and they provide stronger and longer-lasting fillings than can be provided by direct methods. The indirect methods however, are significantly more expensive, both due to the cost of producing the fillings (i.e. equipment needed for such production) and more work needed by the dentist, and at least two visits are required by the patient.

Modified indirect methods have been developed based on pre-fabricated fillings such as described in e.g. US 5,567,156, US 5,697,787, DE 19513568, DE 4123237, and DE 3620542. Such fillings however are typically conically shaped (such as e.g. in US 5,567,156, or as manufactured under the trademark Cerana^{™}) or semi-spherically shaped (US 5,697,787). These shapes are limited by methods to readily prepare cavities to match pre-fabricated fillings. The Cerana^{™} method provides a conical diamond bur to prepare the cavity and specially formed devices are described in DE 19513568 and DE 4123237, to prepare cavities to round shapes of particular diameters. Such specially shaped tools limit the shapes of cavities that are made, and their use requires precision handling and a steady hand so as not to make the cavity too large. SonicSys^{™} fillings are manufactured by Vivadent in different sizes and other shape than conical.

Other modified indirect methods include more effective methods to manufacture solid fillings, that allow the dentist himself to manufacture fillings in his own clinic. Equipment for such manufacturing is sold under the trademark Cerec^{™}, and produced by Sirona GmbH in Germany.

Means for guiding the operation of a dental tool are proposed in US 5,545,039 comprising a probe or stylus fastened to a segment of an apparatus, which segment holds a dental operating instrument such that when the instrument is operated within the mouth of a patient, three CCD cameras record the corresponding movement of the stylus.

The present invention provides an apparatus that significantly reduces both the time and the physical stress of the precision work typically required by the dentist to prepare cavities of a particular shape such as to match pre-fabricated fillings. The invention provides an apparatus that makes possible the control of the function and/or location of a dental operating tool to within a predetermined space.

The dentist is relieved from holding the full weight of a dental tool, but more importantly, the dentist can with the use of the apparatus create accurate pre-defined shapes (e.g. cavities and surfaces for crowns and bridges) without the precision and concentration required by conventional methods which often causes the dentist muscular tension and other physical and mental stress.

### BRIEF DESCRIPTION OF FIGURES

**Figure 1:** An overview of the apparatus illustrated in more detail in Figures 2 to 6.
**Figure 2:** A base with three adjustable tooth fixture grip assemblies.
**Figure 3:** A seat for mounting of a dental tool, 3a: detached tool, 3b: attached tool, 3c: seat opened for mounting of tool.
**Figure 4:** The seat of Figure 3 shown engaged to a sliding member.
**Figures 5 - 6:** The sliding member with engaged seat and tool, joined to a base, also showing a guiding member and guiding shoe for guiding rhe movement of the tool.
**Figure 7:** An example of prefabricated filling (7a), mounted in a tooth in 7b.
**Figure 8:** A digital geometric representation of a tooth.
**Figure 9:** Digital representations of a tooth and a predetermined space within which a dental tool can be operated.
**Figure 10:** An integrated tool and seat connected to a base comprising a frame with three sections.
**Figure 11:** Overview of a preferred embodiment of the apparatus of the invention with a tool connected to a base mounted on a jaw of teeth of a patient.
**Figure 12:** Lower portion of figure shows a preferred embodiment of a base frame, upper portion shows a tool connected to a pantograph and guiding means, for connecting to the base frame.
**Figure 13:** Portion of apparatus (base frame and shoe-holding member, pantograph and guiding shoe not mounted.
**Figure 14:** Exploded view of a lockable joint member for engaged the pantograph and guiding shoe-holding member to the base frame.
**Figure 15 a-c:** Portion of the apparatus for illustrating adjustment of guiding means.

### DESCRIPTION OF INVENTION

The apparatus according to the invention comprises a base, which base is mountable inside the mouth of a subject and in a fixed position with respect to a tooth to be repaired, and a tooth treatment tool, connected to said base wherein the connection between the base and the tooth treatment tool allows for a supported movement of the tool in relation to the tooth, and guiding means for controlling the movement and/or function of said tool to within a pre-determined space. The base may e.g. be a frame, a body, a plate or another construction which is produced in a suitable material and form for insertion and positioning in a fixed position in the mouth of a subject, and allowing a tooth treatment tool to be connected to said base such that the connection between the base and the tooth treatment tool allows for a supported movement of the tool in relation to the tooth. The invention thus relieves the physical stress of holding a dental tool during treatment with said tool.

Preferably, the guiding means for controlling the movement and/or function of said tool, allow manually steered supported movement of said tool within said pre-determined space.

In this context, a tooth treatment tool represents any tool used for dental and/or oral treatment, prophylaxis or diagnosis, including dental turbines, burs, sand blasters, laser beam cutters and water jets; imaging devices such as miniature video cameras or digital (CMOS or CCD) image sensors, also including scanning devices (image, contact and distance sensors) for obtaining 2-D or 3-D image data. In useful embodiments the tooth treatment tool is a turbine or other dental tool which has a powered mechanical or physical action, i.e. where the action of the tool is not merely a result of the actual movement of the tool by a dentist. In the following description the term 'turbine' is to be understood as referring to both high- and low speed handpieces referred to in the art as handpieces, turbines or air-rotors.
The manually steered supported movement is preferably non-path limited, where the term 'non-path limited' in this context refers to movement which is not limited to following any given pre-defined or direction-limited path, rather, the movement of the tool as described herein has at least two and preferably three degrees of freedom, within a pre-determined space as further described below, and can be moved substantially freely within said pre-determined space.

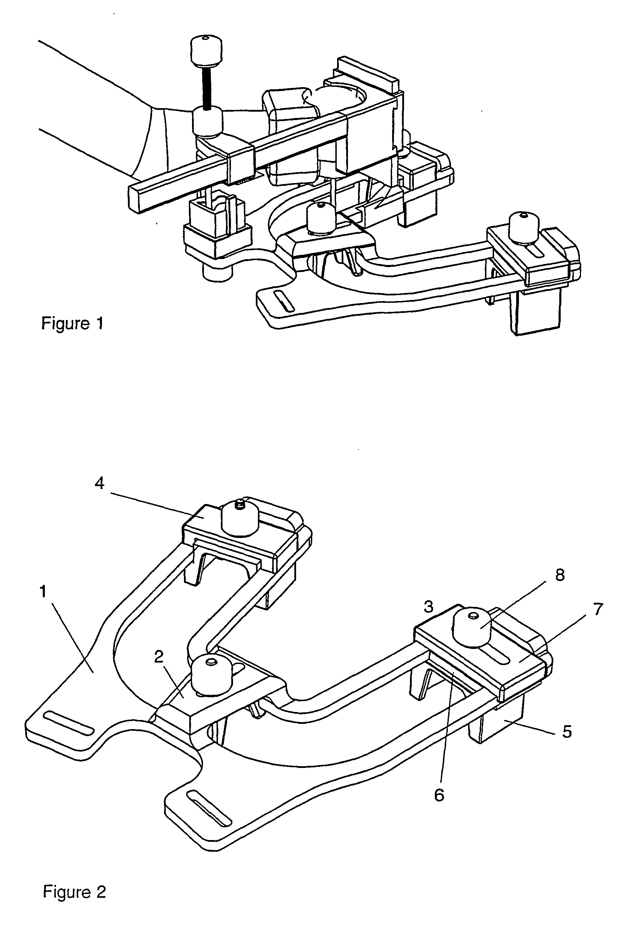

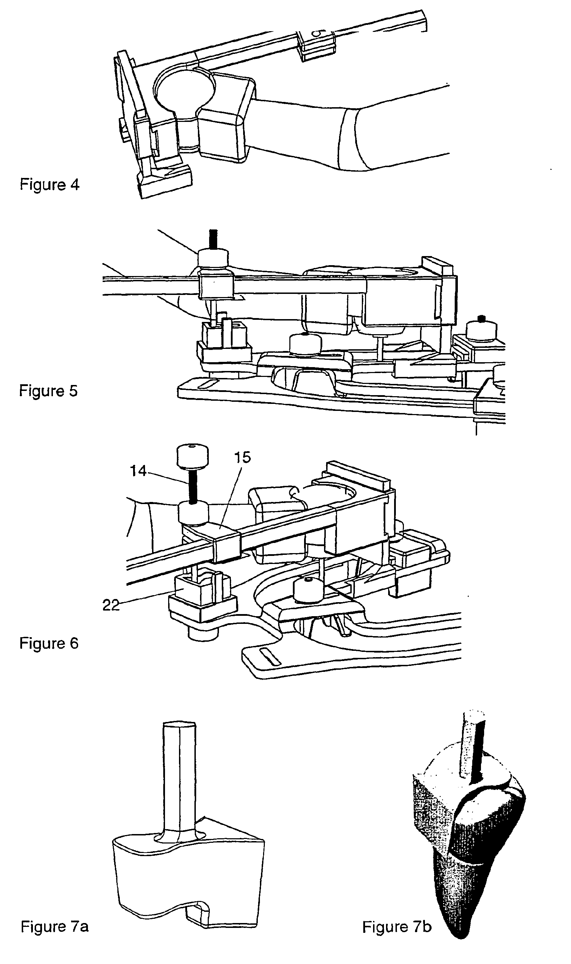

Another embodiment of the apparatus according to the invention has a base comprising at least one section comprising at least one base member which is in proximity to the tooth to be treated when the base is mounted inside the mouth of a patient. A particular embodiment comprises at least one section comprising a pair of parallel members positioned one on each side of a row of teeth when the base is mounted in the mouth of a patient. The parallel members may be in the shape of bars, cylinders, or with any suitable cross-section. In a preferred embodiment the base comprises at least two sections, such as e.g. three sections. In a useful embodiment of the invention the tool is connected to the base through a seat engagement wherein said seat is slidably engaged to said at least one member or pair of parallel members. An example of a base is shown in Figure 2 comprising two sections, each with a pair of parallel members to which a tool seat is slidably connected as shown in Figure 5 and 6. In such an embodiment, a tool can be placed above or in close proximity to a tooth in-between the parallel members of said section.

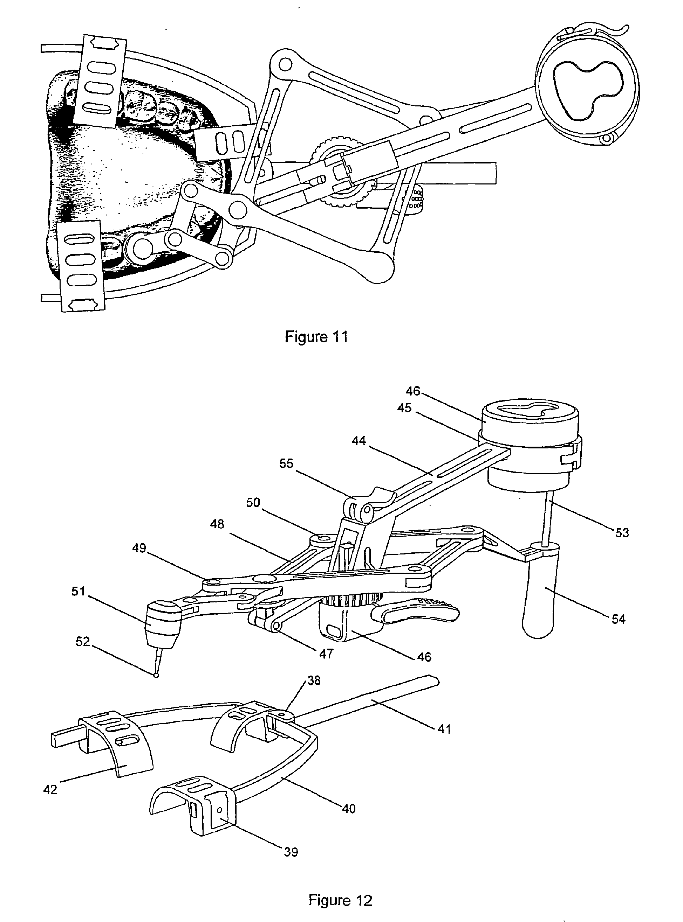

A preferred embodiment of the apparatus comprises a fork shaped frame, wherein a member is shaped such as to fit inside the mouth of a patient, substantially following the arch of the jaw, e.g. by forming a curved "U-shape". A fixed supporting member extends out from the mouth when the base is in place, onto which the tooth treatment tool connects by appropriate connective means. An example of such a base is illustrated in Figure 11 (bottom), showing a frame with flexible holders, which are affixed to the teeth by use of a quick-hardening molding paste such as e.g. Duralay^{™} or Impregum^{™}. It is estimated that three varying sizes of the frame will suit most patients, 2 years and older.

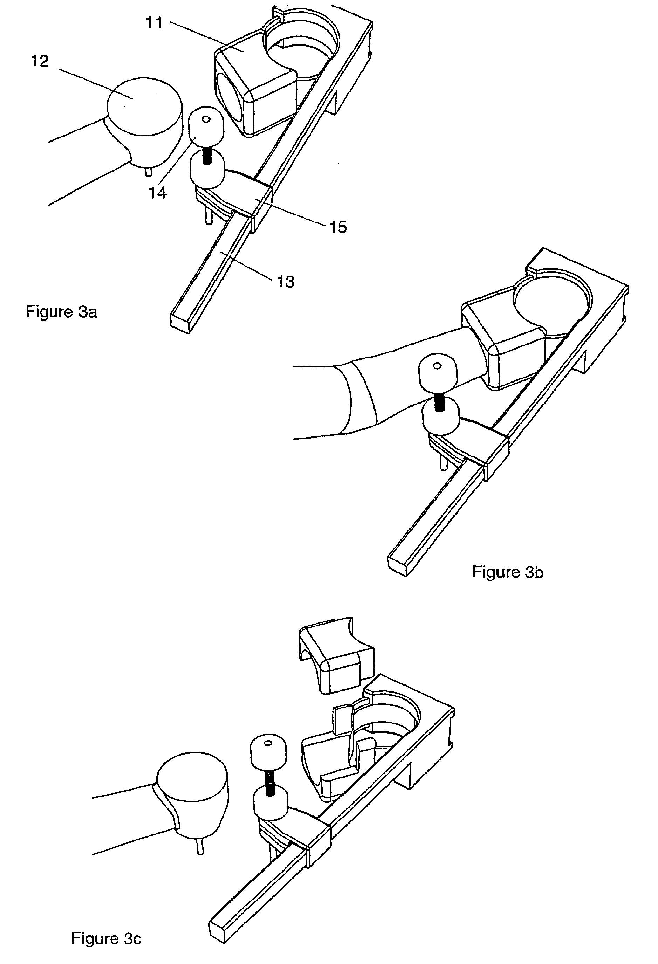

Depending on the particular embodiment of the apparatus, the supported movement of the tool can suitably be a translational movement of the tool in the relation to the tooth, such as preferably a translational movement independently in three directions. Figures 3-6 describe an embodiment that exemplify these features of the invention, wherein a relatively simple mechanical arrangement with low-friction slidable interfaces which allow for movement of a tool with three translational degrees of freedom, after an initial placement of a tool seat onto a frame affixed to the teeth of a patient.
However, in the currently preferred embodiment illustrated by Figures 11-15, the supported movement comprises a combination of both translational movement and angular movement, with three degrees of freedom.

The supported connection comprises in a useful embodiment as mentioned, a seat which is engaged through a bearing unit to a fixed element of the base, and wherein the tool is supported by said seat. Such a bearing unit thus provides the slidable connection to the at least one member of the base, described earlier, and more preferably the bearing unit and seat engagement allows movement of said tool independently along three translational axes.

Figure 3 shows an example of a seat for a tool which is connected through a bearing unit (Figure 4) to a base frame (Figure 5), allowing supported movement of the tool.

In a certain embodiment, the supported movement is further a rotational movement of the tool in relation to the tooth, around at least one rotational axis, such as an axis essentially parallel to the row of teeth comprising a teeth to be treated, such the tooth treatment instrument may reach said tooth at a varying angle relative to the occlusal surface. Such rotational movement may be obtained by a simple pin and hole interface, or preferably by means of an interface with an arch-shaped lip and corresponding groove, e.g. adjacent to a slidable interface for horizontal movement, such that the rotational axis is at or in close proximity to the operating member of the tool such as the bur on a turbine.

The guiding means for controlling the movement and/or function of the tool to within a pre-determined space comprise in useful embodiment a shoe with a guiding space, which shoe is supported by said base, and a guiding member. The guiding member is connected to said tool in a defined geometrically related location to said tool when the tool is connected to the base, and the member is guided by said shoe to thereby control the movement of the tool. The shoe is adjustable to a selected fixed position with respect to the base, and/or optionally the guiding member is adjustable to a selected fixed position with respect to the connected tool, such that the movement of the tool is limited to a pre-determined space corresponding to the guiding space, and such that the pre-determined space is suitably positioned in the tooth to be treated. Controlling the movement is to be understood in this context as limiting the allowed movement such as to a pre-determined space, and not necessarily effecting the movement within that space. The term shoe refers in this context to a body of any suitable outer shape made from any suitable material, to define the guiding space within the shoe.

One example of said geometric relation is simply that the guiding member is in a fixed position to said tool when the tool is connected to the base.
In an embodiment such as demonstrated on Figure 6, the shoe comprises a body with a model cavity essentially defining said pre-determined space. It is critical for such a described functionality to precisely position the shoe and/or guiding member for the correct position of the pre-determined space, which determines e.g. the material to be removed. This may be accomplished by fine-adjustment of the shoe with the tool and the tool guiding member in place, e.g. be placing the guiding member at the edge of the guiding space and adjusting the position of the shoe such that the operating member of the tool (e.g. a turbine) is at the corresponding edge of the pre-determined space to be cleared. For accuracy of the guided movement, it is highly preferable that the tip of said guiding member has essentially the same width as the tip of the operating member of the tool (e.g. tip of a turbine). Preferably, the engagement of the shoe to the base allows for such fine-adjustment in three independent directions. Alternatively, the position of the guiding member is fine-adjusted after approximate positioning of the shoe such as e.g. by sliding the shoe in a shoe seat along parallel members of a frame comprised in the base as described above. Figure 6 shows a useful embodiment wherein the shoe is slidably adjusted in one direction in a shoe-mounting section of the base frame, and the guiding member is adjusted in two directions, thereby determining the exact location of the pre-determined space for the tool to operate in.

A selection of shoes with different guiding spaces is further provided to allow for suitable different sizes and shapes of the predetermined space. An important utility of the invention is the possibility to create cavities of a pre-determined size and shape, into which a pre-fabricated filling fits. Thus, a dentist may after inspection of a tooth to be treated, select a suitable filling and its corresponding guiding shoe to create a cavity for the filling.

Shoes are also provided for guiding the shaping of tooth stumps for the insertion of dentures and artificial teeth, including crowns and bridges. Preparation of a tooth stump typically involves creating a flat surface just below the gum line and leaving a stump of a particular shape (e.g. semi-conical or boss shaped) which fits a corresponding hollow in the crown. A shoe for such shaping will thus have a guiding space with a flat bottom with a protrusion corresponding to the desired stump shape. Said bottom will need to be at least the size of the cross-section of the tooth at the created surface; the guiding space of such a shoe may further comprise edges limiting the operating space such as to protect adjacent teeth. Optional protection (both in preparation for dentures and in creating cavities) may be obtained by placing a thin foil (e.g. of thin, flexible stainless steel) between the tooth to be treated or removed and an adjacent tooth.

Preferably, for such controlled movement of the tool, the operating member of the tool may be operated essentially in a vertical position, or at a selected fixed angle to the occlusal surface.

It should be noted that the defined geometric relation between the tool and guiding member may also include other implementations than the above described fixed relation. In a particularly useful embodiment the tool and guiding member are connected via a pantograph. In one such embodiment, the tool is connected to the base by a pantograph which holds the guiding member, which pantograph may be connected to the frame via a pivotal joint on a joint member or through other engaging means, such that the pantograph is adjustably lockable to the base. In such an embodiment, the guiding means suitably comprise the aforementioned guiding means comprising a shoe and guiding member, wherein the model cavity of said shoe is scaled up by a pre-determined ratio compared to the pre-determined space, and wherein said pantograph scales down the movement of said tool compared to the movement of said guiding member by the same ratio such as to guide said tool to within said pre-determined space.
This is illustrated in Figure 11 showing an overview of an apparatus of this type, with more detailed illustrations in Figures 12-15. The apparatus comprises a base shown mounted on a jaw of teeth, with a joint member (43) lockably connected to a support member (41) of the base. A pivotal joint (61) connects a joint connection (47) to the joint member (43), holding the pantograph (48) which on one end holds a tool (51) such as a turbine, and on the other end (the farther end) holds the guiding member (53). The pantograph itself is a generally well known device, the ratio of the pantographic front and rear rhombi defined by the placement of the central joint on the pantographic crossed arms define the ratio of the movement of the far ends of the rhombi. In one embodiment the pantograph is designed such that the tooth treatment tool follows the mirror image of the movement of the guiding member, but scaled down by a factor of three. This factor, may however vary depending on the design of the pantograph, but is suitably in the range of about 2-5, such as about 3-4, e.g. about 3.

Another useful embodiment of the apparatus comprises guiding means of a different type, comprising a computer system storing a digital geometric representation of said predetermined space, means for registering the movement of said tool and input such movement data within said computer system after having placed the tool at a suitable starting point corresponding to a reference point of said pre-determined space; means for controlling the location and/or function of said tool to within said predetermined space, by stopping the movement and/or function of said tool when the location of said tool reaches the boundary of said predetermined space.
In essence, this embodiments relates to the same general principle as previously described above, except that the shoe and guiding space are defined digitally by means of said computer system, and the guiding member is replaced by means of registering the movement of the tool (e.g. by displacement sensors) and inputting such movement data within said computer system which is programmed to give a signal when the tool reaches the boundaries of said predetermined space, and said signal either stops the movement of the tool in a direction outside said boundaries, or stops the function of the tool (turns off tool driving mechanism, which can be e.g. electric power, laser light, or compressed air stream).

In another embodiment, the means for controlling the location and/or function of said tool comprises: an imaging device for obtaining data of the tooth to be repaired, wherein said data provides positional information (and preferably topographical information) of said tooth with reference to said base; means for determining the location of said tool in relation to the tooth to be repaired; a computer system which can import said data to produce a digital geometric representation of said tooth with positional coordinates in reference to said base, said system interacting with said location determining means, and interacting with said tool to control the location and/or function of said tool in relation to said tooth to a pre-determined space. Such an imaging device is any applicable imaging or scanning device (both referred to herein as imaging devices) which can be operated inside the mouth of a patient for obtaining useful image data of the tooth to be repaired. Useful data in this context is defined as data providing positional information of said tooth essentially defining the surface and boundaries of said tooth.

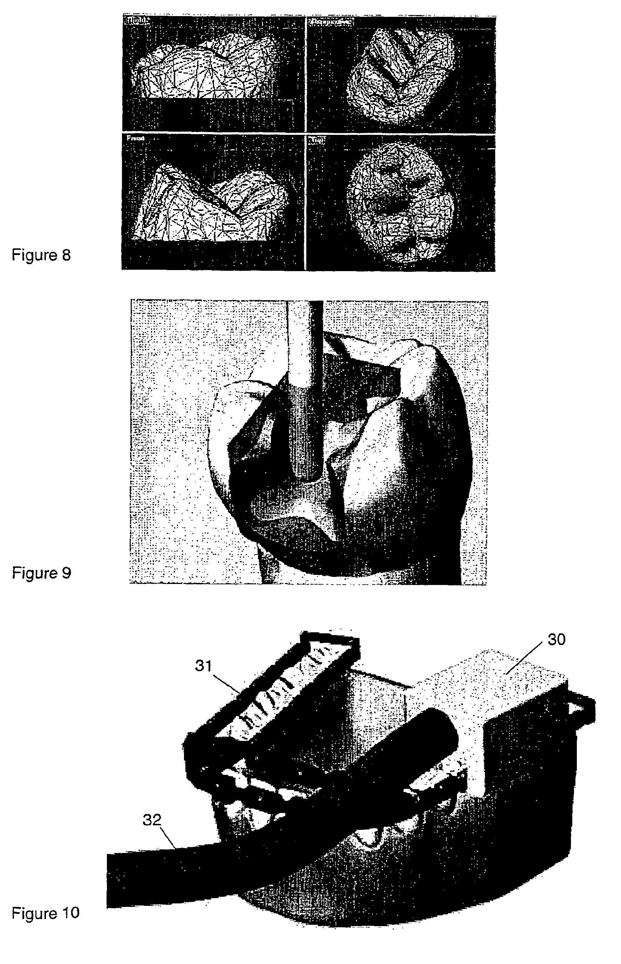

In an advantageous embodiment, the imaging device comprises an image detector supported by said base. The detector may e.g. be shaped in such a way as to be interchangeable with the treatment tool in said seat. In such a way, said means for determining the location of the tool will likewise provide the location of the image device. In a particularly useful embodiment the image detector is a contact sensor, such that said image data is collected by scanning the surface of said tooth with the contact sensor. In such scanning the sensor and means for determining the location of the tool or image device interact to provide the positional coordinates of the sensor in contact with (or in known distance to) the surface of a tooth. Such information is gathered with a high enough frequency to provide sufficient density of points to define with sufficient precision the surface of said tooth, as exemplified in Figure 8. In such a way, a geometric representation of the tooth is obtained with positional information in reference to the base (and thus the tooth to be treated, as the base is fixed with respect to the tooth) and the location of the treatment tool can be precisely determined in reference to said geometric representation and thus the tooth.

Preferably, the computer system provides information regarding the location of the tool in relation to the tooth, by means of the geometric model. In a useful embodiment, the computer systems displays the geometric model on a monitor, and preferably it provides the location of the tool on said monitor, in relation to said tooth which is represented by the geometric representation of the tooth.

In a further embodiment, the computer system generates a geometric representation of said pre-determined space within the tooth to be repaired, which representation may optionally be superimposed on the geometric representation of the tooth or teeth to be repaired. Such superposition is suitably done with a boolean subtraction of the geometric representation of said pre-determined space from the geometric representation of the tooth be treated, to obtain a geometric representation of the tooth with a cavity to be prepared, defining an operating space. The operating space is thus limited by the tooth material which is not to be removed, i.e. material outside the pre-determined space, in the tooth or teeth to treated, and in adjacent teeth. An example of an image showing a geometric representation of a tooth, an operating space defined by a pre-determined shape of material to be removed, and the surrounding tooth material, and a representation of a tool, is shown in Figure 9.

The location determining means for determining the location of the tool - and in some instances of the image device, as discussed above - are of any suitable kind known to the person skilled in the art, which will provide sufficient precision needed for guided dental work, and on a small enough scale to be part of equipment used inside the mouth of a human subject. Such precision is on the order of ±0,1 mm or less, preferably within ±0,05 mm or less, such as within ±25 µm or less. A high precision in such location determination may be obtained with the use of inductive displacement sensors. These are manufactured small enough to be compatible with an apparatus according to the invention, and are suitably placed at the interfaces of the slidable members of the base and/or seat. The measuring principle is based on a segmented coil contained in a rod-shaped housing and when supplied with an alternating current, the coil induces eddy currents in a measuring ring, the currents in turn influence the coil sections and the voltage drops of the individual segments can provide linear output signal that is proportional to the position of the measuring ring. Such inductive displacement sensors are manufactured e.g. by Micro-Epsilon^{™}, Germany.

Another useful type of location determining means are based on the use of an external location member similar to the guiding member described above, except the location member has two laser diodes at or near its end, and two spatial light detectors (e.g. CMOS or CCD) which are placed at an angle in proximity to the end of the location member detect the light emitted by the diodes and thus provide the movement of the location member and the identical movement of the tool.

When provided with the geometrical representation of the tooth and the operating space which the tool is to be operated in, and further provided with the location of the tool in relation to said geometric representation, the computer system with the imported geometric representation and interacting with the location determination means further interacts with the tool to control the location and/or function of the tool to a pre-determined space.
In one embodiment this is achieved by stopping the operation of the tool when it reaches a boundary of the operating space, e.g. by regulating the electrical power driving the tool, or - as in the case of a turbine - by stopping the airflow driving the tool, or stopping the function of the tool by applying friction to the moving operating member of the tool through a break mechanism interacting with said computer system.

In the case where the tool is a dental turbine, the turbine is in certain embodiments a conventional dental turbine and the said seat is shaped to firmly support said turbine. In this context, firm support is to be understood such that the operating member of the tool is in a fixed position with respect to the seat, and the positioning of the tool in the seat further needs to be accurate such that the position of the operating member of the tool with respect to the seat is always the same, such as after disconnection and reconnection of the tool and seat.

In other embodiments, the tool is specially constructed for operation according to the invention, e.g. by not having a conventional handle for holding the tool, but merely a lighter and smaller handle for guiding of the tool. The handle may further have a joint such that when moving the tool and seat upwards, less of a torque will affect the part of the tool joined to the seat, if a torque is unintentionally applied to the handle.

In yet a further embodiment of the apparatus, the supported movement of the tool is remotely controlled movement. Such movement is suitably affected by micro-actuators such as micro-motors, hydraulic or pneumatic systems, interacting with said computer system. The apparatus may further comprise a user interface, such as a mouse, joystick or keyboard, to steer said movement. The micro-actuators are suitably comprised in the seat engagement connecting the tool with the base. In a preferred embodiment the tool is integrated within the seat and bearing unit engagement, as shown in Figure 10. Thus, for tooth treatment such as the removal of tooth material, the computer system will store collected image data and create and store a digital geometric representation of the tooth, subtract a pre-determined space corresponding to tooth material to be removed to create a new geometric model (such as shown in Figure 9) which defines the space within which the operation of the tool is allowed. The computer system further receives positional information from location sensors within the seat and/or bearing unit and will override the input signal steering the movement of the tool when the operative member of the tool reaches a boundary of the operating space. (Normally, the user interface is connected to the micro-actuators trough the computer system.)

A further embodiment uses such micro-actuators for effecting the movement of the tool, wherein the micro-actuators interact with the computer system and the computer system steers the movement based on the pre-determined space. The computer system in this case uses a program which uses the digital geometric model defining the operating space to calculate a suitable path that the tool is moved by, by the micro-actuators when an operator (i.e. the dentist) runs the program.

In yet a different embodiment of the apparatus according to the invention, the tool is connected to said base through a remote connection. The remote connection preferably comprises an electromagnetic radiation source joined to said tool which source emits one or more beams, and a spatial absorption detector, such as a CCD or CMOS detector, comprised in said base, which detector detects spatially the radiation emitted by said source, and wherein the spatial detection signal from said detector is forwarded to said computer system to determine the location of said tool, in relation to the base and thus the tooth being treated.

The invention further provides in a related aspect a base, e.g. such as described above, for mounting inside the mouth of a subject, for support of a dental treatment tool. In the currently preferred embodiment said base comprises a fork-shaped frame comprising two symmetric distal members with substantially parallel distal ends connected by a proximal member which is further connected to a support member which extends out of the mouth of the subject when the frame is mounted within the mouth of said subject; at least two but preferably three adjustable (e.g. slidable) holders mounted on the frame, which holders are suitably shaped such as to form fixtures holding the base fixed to a jaw of teeth, when introducing suitable molding paste inbetween the holders and teeth of said jaw and allowing said paste to harden. "Substantially parallel" means that the distal ends can be essentially parallel or at a narrow angle, such as of less than 35°, e.g. less than 15°.

In a useful embodiment, said proximal member has a hinge for varying the angle between the distal members.

Said holders preferably have a substantially curved or angled shape, allowing sufficient contact area with said molding paste, to form fixtures to said teeth when said paste has hardened, keeping the base in a fixed position.

The holders may in particular embodiments be lockably slidable e.g. by a spring-release mechanism such that each holder comprises a simple push button for releasing the locked-in position of the holder on the frame member onto which it is mounted.

A method is provided for treatment of a tooth in a patient, such as for removal of tooth material, comprising the steps of: arranging a base inside the mouth of the patient in a fixed position with respect to the tooth to be treated; connecting a tooth treatment tool to the base, wherein the connection between the base and the tooth treatment tool allows a supported and preferably non-path limited movement of the tool in relation to the tooth; performing the treatment by supported movement and operation of the tool. The tool is any dental tool as described above, and in useful embodiments comprises a dental turbine, and the base is preferably as described above. The tool is preferably connected with a seat and bearing unit assembly as described above, such as with a sliding member for slidable movement along a member of the base, as described above. However, in presently preferred embodiments, the connection between the base and the tooth treatment tool comprises a pantograph device holding the tool at one end, and a guiding member at the other end, wherein the guiding member is in contact with a guiding shoe adjustably lockable to the base, to thus allow the supported movement of the tool on relation to the tooth, for performing the treatment according to the method, by supported movement and operation of the tool in a pre-defined space within the tooth to be treated, which pre-defined space is defined by the guiding space of the guiding shoe.

It will be appreciated that the method, is particularly useful for removal of tooth material from a tooth of a patient, wherein the method comprises the steps of:
determining at least one characteristic of said tooth, such characteristics being location and approximate shape of tooth material to be removed;
determining - based on the determined characteristic of the tooth, the size and shape of an appropriate space - *operating space* - within which a tooth treatment tool as defined above is to be operated;
arranging a supporting base such as any of the above described, inside the mouth of the patient and in fixed position with respect to said tooth;
connecting said tool to said base such that said connection allows for supported movement of the tool, allowing the operation of said tool within said operating space; preparing the tooth with the use of said tool essentially to clear the pre-determined operating space.
The connection of the tool to the base is preferably such as described above.

The method comprises repairing tooth decay in a tooth such as removal of caries, wherein: said characteristics being location and approximate size of the decay to be removed; said appropriate space - *operating space -* is determined such that an appropriate filling may be inserted into said space; and wherein the method further comprises the steps of: optionally applying fastening means such as dental cement into said cavity and/or onto the filling to be inserted into said cavity; and inserting said filling into said operating space; and optionally finishing the surface of said filling. This method thus allows a dentist to readily and quite quickly prepare a cavity into which a pre-formed filling will fit, e.g. a pre-formed porcelain or plastic filling. Note that the terms dental fillings and dental inserts are used herein interchangeably. The use of such pre-made fillings has been severely limited due to the difficulty of preparing cavities of exact size and shape to fit the filling, with currently available prior art methods.

An appropriate filling is selected such that it will fit in a cavity created such that all caries is removed and the amount of healthy tooth tissue material removed is minimized.

Preferably, during said preparing, the location and/or function of said tool is controlled to within the pre-determined *operating space*, with an apparatus as described above.

Such controlling of the location of the tool may in a useful embodiment of the method be effected by adjusting and fastening a shoe with a guiding space onto said base such that when a guiding member -which is fixed, or in a non-fixed but geometrically defined related position, relative to the connected tooth treatment tool - is inserted in the guiding space, the location of the operating member of the tool is limited to within the operating space. This is accomplished with an apparatus as described above.

The location and/or function of said tool is controlled by the steps of:
- obtaining topographical data of the tooth to be treated, wherein said data provides positional information of said tooth with reference to said base;
- importing said data into a computer system and producing a digital geometric representation of said tooth with positional coordinates in reference to said base;
- entering information defining the shape to be removed into said computer system;
- obtaining a geometric representation of said tooth after subtracting the shape to be removed essentially defining said operating space, and storing in said computer system;
- connecting said tool with said computer system;
- determining the location of said tool during said treatment with location determining means, and providing the computer system with data defining the location of the tool; and
- performing said preparing, wherein the computer system controls the function of said tool to within the operating space.
The method is suitably performed with an apparatus as described above.

The preparing step is accomplished by remotely controlling motorized movement of said tool through a user interface interacting with said tool, e.g. with an apparatus as described above.

Where the method is used for repair of tooth decay, the appropriate preformed filling may suitably be fabricated such that the periphery and inclination of said filling essentially matches said operating space, i.e. the exposed surfaces of the filling need not be perfectly aligned with the surfaces of the tooth as the filling may be finished in a further optional step of the method with any appropriate finishing means well known to the person skilled in the art such as a turbine, an ultrasonic bur, or a file.

The filling may be fabricated such that its shape essentially matches the portion of the operating space which is within the tooth, with the provisio that the filling may optionally protrude above and/or beyond the original surface of the tooth, and with the provisio that the space within the tooth may optionally extend deeper than the filling. i.e., after removal of infected tooth tissue, it is not necessary to fill up the entire prepared cavity, and as mentioned above the surface of the filling may conveniently be finished after insertion.

It is a particularly noteworthy feature of the method that a *pre-fabricated* filling may be selected based on the size, shape and location of said tooth decay, and said operating space is then pre-determined such that the periphery and inclination of said operating space essentially matches the periphery of said filling. The invention thus offers the possibility for a dentist to have a stock of pre-fabricated fillings of the most commonly needed shapes and sizes (it is estimated that only 9 different fillings would be suitable and sufficient for the repair of about 90% of caries infections which appear in molars (Ericsson, Yngve et al., *Kariologiska principer,* 1980, Lagerblads AB).

Figure 7a shows an example of a pre-fabricated filling having a pin for ease of handling, and in Figure 7b such a filling is shown inserted in an appropriately shaped cavity in a tooth, before finishing of the filling has been performed.

The inventor has found a simple and time-saving method for finishing of such pre-fabricated fillings, which relieves the dentist from performing the operation on the filling inside the mouth of the patient. Briefly, after forming the desired cavity and examining that the selected fillings fits in the cavity, the dentist leaves the filling in the cavity (having not added any adhesive cement). He thereafter forms a mold of a the tooth with the inserted non-finished fillings and the adjacent teeth, with molding dough suitable for dental work; removes the mold such that the filling adheres to the mold and stays fixed in the mold; fills the mold with quick-hardening model substance (optionally after blocking the substance from filling but the mold corresponding to prepared tooth); removes after hardening the model of the teeth with the filling inserted in the model of the treated tooth. He can then finish the filling with suitable means to obtain a desired surface on the filling, while the filling is inserted in the model of the tooth, and thereafter insert it in the prepared cavity with adhesive cement.
Finishing of an inserted filling and/or prepared tooth surface may be performed by the tool of the apparatus as described above, wherein during said finishing step the active member of said device may optionally be exchanged for an appropriate finishing-active member, such as a bur or file. When the location and/or function of the tool is controlled by the use a digital geometric model and computer system as described above, such finishing may suitably be performed such that the function of said tool is limited to a space determined by the surface of the geometric representation of the tooth, prior to the treatment, with similar methods and means as described above. Essentially, a new operating space is determined for this step, which is defined by the surface of the tooth as it was prior to the treatment, or optionally by another desired surface.
The invention additionally provides in related aspect a pre-fabricated dental insert for use in the method. The insert is as described above, preferably of a conventional ceramic material.

A dental insert system comprising a pre-fabricated insert such as have been described above, and a guiding shoe defining a guiding space, also as described above, wherein the shape of the insert substantially matches the shape of the guiding space, such that the insert will fit into a prepared cavity when using a dental tool guided by the guiding space are described.

Such a dental insert system is particularly useful with an apparatus of the present invention when performing the methods described herein for dental repair.
The dental insert system comprises a plurality of differently shaped inserts and a corresponding plurality of guiding shoes matching said inserts, i.e. matching pairs of insert and shoe.

A guiding shoe for dental repair, which guiding shoe comprises a body with a guiding space, which guiding space is shaped so as to define the shape of a cavity to be prepared in a tooth, for inserting a pre-fabricated insert is described. The guiding shoe is intended for use in combination with an apparatus such as of the current invention, and is preferably such as has been described above, and in the and is further described in the following detailed description.

It should be noted that though all aspects of the inventions will be most useful in the treatment of tooth in human subjects, it is within the general scope of the invention to adapt the invention for other animals in need of tooth treatment, particularly other mammals such as dogs, and various zoo animals.

### MORE DETAILED DESCRIPTION OF THE PREFERRED EMBODIMENTS WITH REFERENCE TO THE FIGURES

The base of Figure 2 comprises a rigid frame (1) forming two sections, each with two parallel members, and with three adjustable tooth fixture grip assemblies (2,3,4), one (2) centered between the two sections and the other two grip assemblies (3,4) are placed one on each frame section. The frame is placed in a fixed position onto the occlusal surfaces of the dental arch. (The orientation refers to a lower jaw and is reversed in case of an upper jaw.) The tooth fixture grips can be adjusted in to allow fixture of the frame to teeth of varying size and jaws of varying width and angle. Fixture (3) allows for adjustment in two directions and comprises a holder (4) with a threaded bore, a centerpiece (5), a frame grip (6) and a fixing screw (7). The holder (3) can be adjusted in two directions parallel and perpendicular to each frame, as the fixture assembly is slidable on the frame, and the holder (5) and fixing screw (7) are slidable (about ±5 mm) in the slits of the centerpiece (5) and frame grip (6). Fixture (4) does not have a centerpiece and thus the frame may be brought closer to the teeth on that side, or just below the occlusal surfaces. Preferably, fixture (4) is placed on the side of the arch containing the tooth to be treated as that allows a bur of a turbine to reach further down. The center-fixture (2) is also adjustable. With a base such as shown, three frame sizes will suffice to cover close to all (~99%) patients. The frame is mounted in the mouth of a patient and the holders adjusted such they will fit on opposite teeth in the jaw, and leaving access to the tooth or teeth to be treated. The holders can be adjusted in two directions,. After correct adjustment the assemblies are fastened by tightening the fixing screws. After adjustment and fastening of all three assemblies, quick-hardening molding paste such as, e.g. Duralay^{™} or Impregum^{™} is injected in between the holders and the supporting teeth underneath and allowed to harden and thus fixing the base to the dental arch. (inserting the paste may simply be done by removing the base, filling the holders with suitable amount of paste and placing the base back in the same position in the mouth of the patient.)

The frame sections may be in one plane as shown but in a preferred embodiment, designated frames for the lower dental arch have slightly inwardly tilted sections as the molars of the lower jaw are slightly tilted in such a fashion.

Figure 3a shows a seat (11) for mounting of a dental tool such as turbine (12). The seat is shaped to firmly support a tool of a specified shape, in this embodiment the seat allows for a 60° rotation of the tool handle (for ease of operation and to improve access of the tool) but the operating member of the tool (such as a diamond bur of a turbine) is always in a fixed position with respect to the seat.
Attached to the seat is a bar (13) holding a guiding member (14) for control of the movement of the seat with the tool, when the seat is connected on to the base. The guiding member (14) is threaded for vertical adjustment and fits in to a threaded bore in the lower jaw a clamp (15), slidably adjustable to the bar. After adjustment of the height of the guiding member and position of the clamp, the tightening screw is tightened down which locks the height of the member and locks the clamp on the bar. Figure 3b shows a dental turbine inserted in the seat, and Figure 3c shows the seat opened for insertion of a tool

In figure 4, the seat is shown engaged to a sliding member (20) which in turn is slidably joined to a frame mount. The frame mount is slidably mounted on the parallel members of a section of the frame in Figure 1.

The frame mount is slidably mounted on one section of the frame of the base, shown in Figures 5-6. A guiding shoe (22) is mounted onto the base and the guiding member (14) is inserted in the guiding space (23) of the shoe, thereby limiting the movement of the tool to within a pre-determined space corresponding to the guiding space.

Adjustment of guiding member and guiding shoe: By adjusting the position of the clamp (15), the height of the guiding member (14) and the position of the guiding shoe (22), the exact location of the pre-determined space is set. The adjustment is suitably done by, e.g. placing the tool where an edge of the prepared cavity should be and adjusting the guiding member and guiding shoe such that it contacts the corresponding edge of the guiding space of the shoe. The guiding member has essentially the same diameter as the operating member of the tool such that the position of all edges of the operating member will correspond to the corresponding edges of the guiding member.
Another embodiment is illustrated in Figure 10, as an integrated tool and seat (30) for connection to a base comprising a frame (31) with three sections. (Fixtures supporting the frame by contact to the are not shown) The supported movement of the tool is motorized movement steered by a user input device (not shown) and controlled by a digital geometric model of the tooth to be treated and a pre-determined space within which the tool can be operated, as shown schematically in Figure 8. An integrated cable (32) holds a power cord to power the motors, a transmission line for transmitting input signals for steering the tool, location signals from location determining sensors, and optionally pressurized air to power an air-driven turbine.

Figure 12: The lower portion of the figure shows a preferred embodiment of a base frame, as described above, comprising a fork shaped frame (40) shaped such as to fit inside the mouth of a patient, substantially following the arch of the jaw. A fixed supporting member (41) extends out from the mouth when the base is in place. Flexible holders (42) which can be affixed to the teeth by use of quick-hardening molding paste, are slidably mounted on the frame (40). When providing sufficient amount of molding paste in the space between the holders and adjacent teeth, the hardened paste and holders create rigid fixtures keeping the base in a fixed position relative to the teeth. A lockable joint member (43) slides onto the supporting member (41) allowing lockable adjustment of a shoe-holding member (44) which on one end has a fixture (45) for a guiding shoe (46), and on the opposite end has a joint connection (47) which connects a pantograph (48) to the shoe-holding member (44). The shoe-holding member (44) is fastened on a pivot member (61) by a quick-release mechanism (55).
The pantograph (48) holds on one end a dental tool, illustrated here as a dental turbine (51) and on the other end a guiding member (53). The joint connection (47) and the pantographic joints (49, 50) allow essentially free movement in three directions of the dental tool (i.e., a combination of translational and angular movement, with total of three degrees of freedom).
By this arrangement, the dental tool stays in a defined geometrically relation to the guiding member, defined by the pantograph design (ratio of length of front and rear pantographic rhombi). In the embodiment shown, the guiding member has an extended joystick handle (54), by which an operator of the instrument (the dentist or dental assistant) steers the movement of the tool (51). The movement of the tool's active member (52) essentially follows the mirror image of the movement of the endpoint (56) of the guiding member (53), but scaled down by a factor determined by the design of the pantograph. As shown in the illustrated embodiment, the pantographic factor is about 3. For improved precision, the width of the guide member endpoint (56) should correspond to the width of the active member (52) of the tool, multiplied with the pantographic factor.

### Adjustment of the position of the guiding shoe:

The guiding member (53) can be locked in a certain position by the guiding shoe (such as at the far edge of the guiding space within the shoe, e.g. by a simple hook mechanism (57); then, by loosening the joint member (43) and the pivot member (61), movement of the joystick handle (54) will move not only the tool and guiding member but also the shoe-holding member (44) and guiding shoe (46). When the tool's active member (52) is suitably positioned at a suitable point location by a tooth to be treated, i.e. the point of the edge of the desired operating space, which corresponds to the point of the edge of the guiding space at which the guiding member is positioned, the shoe-holding member (44) is locked in place by fastening the joint member (43) and pivot member (61). Thereby, the guiding space of the guiding shoe now defines the desired operating space within a tooth.
Adjustment of the pivot member (61) further allows suitable adjustment of the angle of tool, before the pivot member is locked in position. The quick-release mechanism (55) allows removal of the shoe-holder and pantograph, without changing the positional and angular adjustment of tool relative to the base.
By exchanging the tool for a positioning plate tool (a tool with an affixed plate perpendicular to the tool direction), the angle of the tool can be readily adjusted to be perpendicular to the occlusal surface of the tooth to be treated.

### Operation of the apparatus of Figure 12, for insertion of a pre-formed dental insert:

A base with a suitably sized frame is selected to match the jaw size of the subject to be treated. The base is mounted in the subject by substantially filling the space underneath the curved flaps of the holders (42) with quick-hardening dental molding paste, and the holders are adjusted on the frame such as not to block access to the tooth to be treated. The frame with paste-filled holders is pushed on to the jaw with the tooth to be treated and the paste is allowed to harden, thus fixing the base in relation to said tooth. Judged from inspection of the tooth, a guiding shoe is selected with a guiding space matching a selected suitable pre-formed insert, and the shoe is inserted in the shoe-holder (45). The joint member (43) is slid onto the support member (41) with the shoe-holding member (44) and pantograph (48) mounted. The guiding member (53) is set in locked position (by means of locking mechanism (57)) at the outer edge of the guiding space and the position of the tool (51) adjusted such that the active member of the tool (52) is located at position by said tooth corresponding to the farther edge of the cavity to be made, i.e. the point of the cavity to be made corresponding the locked position of the guiding member. Typically, the height of the height ring (64) and the angle of the pivot member (61) are preferably adjusted such the angle of the tool is parallel to the axis of the tooth when the active member of the tool is at the mid-height of the cavity to be prepared. After positioning and adjustment of the tool, the tool is turned on and operated by moving the joystick handle to fully clear the pre-determined space of the tooth. For added safety and guiding, it is useful to insert a thin metal foil between the tooth to be treated and the next tooth adjacent to the side of said tooth being treated. The insert can then be inserted into the prepared cavity, typically after application of a thin layer of dental cement. The insert is finally finished, by conventional finishing means and/or by using a suitable finishing tool inserted in the apparatus.

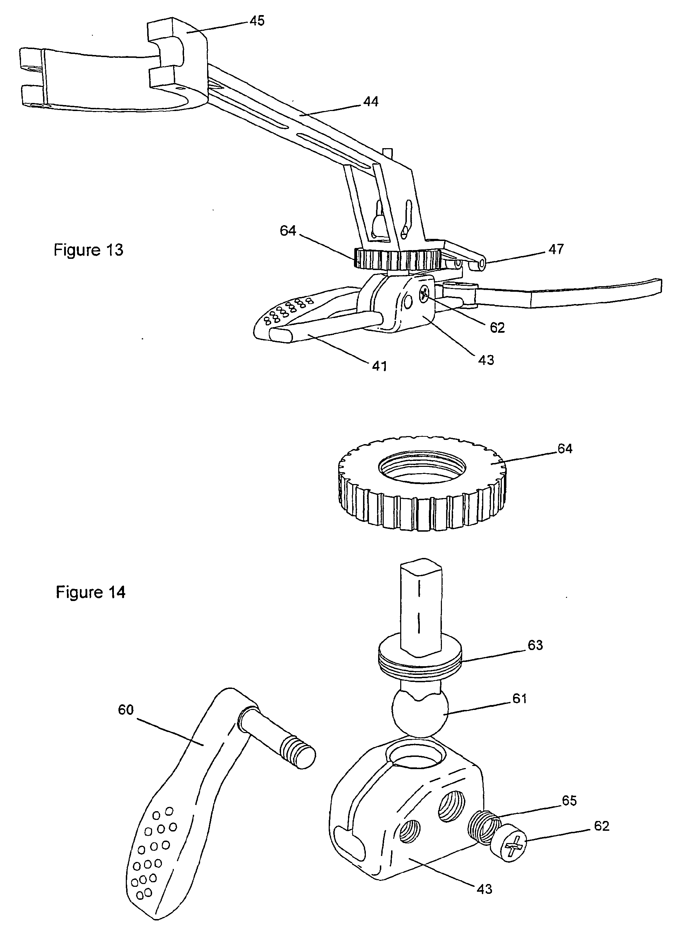

Figure 13 shows the base (40, 41) and shoe-holding member (44) in a rear view, as compared to Figure 12, with the pantograph and guiding shoe not mounted. Only half of the shoe fixture (45) is shown, into which the guiding shoe may be locked.

An exploded view of the lockable joint member (43) is shown in Figure 14 showing a locking screw handle (60) which locks the slidable joint member (43) onto the supporting member (41) of the base (not shown); a pivot member (61) which is lockable in a selected position by tightening of a locking screw (62). A threaded height ring (64) is screwed on a threaded portion (63) of the pivot member (61), for adjusting the height at which the shoe-holding member (44) is mounted on the pivot member. Thus by suitable adjustment of the height ring (64) and the angle of the pivot member (61), a suitable operation angle of the tool (51) at a given position is set.

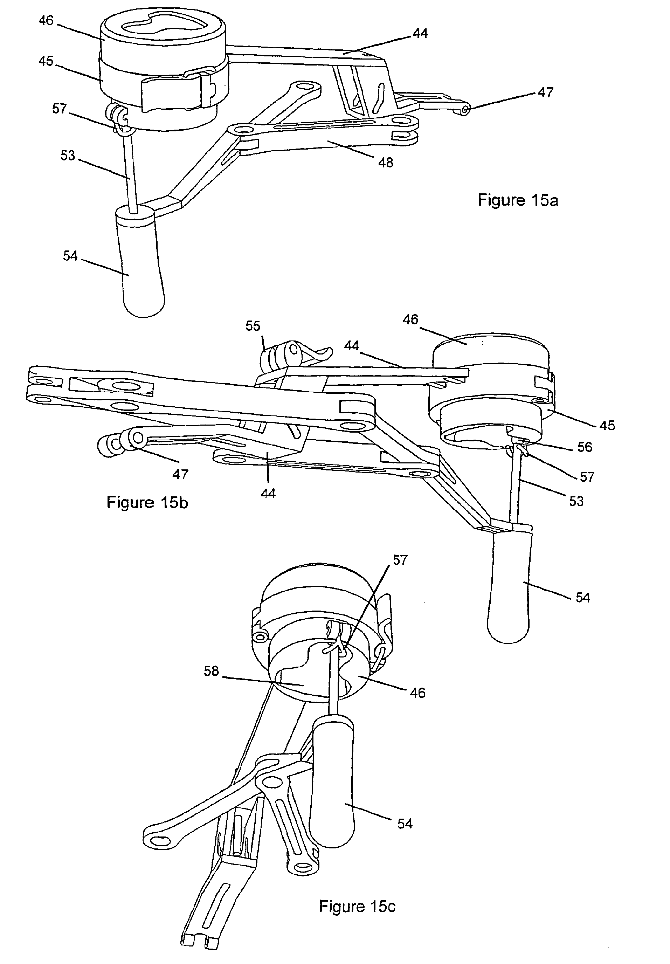

Figure 15 gives a view of a portion of the apparatus, illustrating the locking mechanism (57) and showing the guiding space (58) of the guiding shoe (46).

## Claims

1. A tooth treatment apparatus comprising:
a) a base (40) having means for fastening the base inside the mouth of a patient and in a fixed position with respect to a tooth to be treated,
b) a tooth treatment tool (51), connected to said base in a manner allowing supported movement of the tool in relation to the base,
c) guiding means (48,53) for controlling the movement and/or function of said tool to within a pre-determined space.

2. The tooth treatment apparatus of claim 1, wherein the guiding means allow manually steered supported movement of said tool within said pre-determined space.

3. The tooth treatment apparatus of claim 1, wherein the guiding means comprise:
I. a shoe (46) with a defined guiding space (58) which shoe is connected to said base (40) adjustable and lockable in a selected fixed position in relation to the base;
ii. a guiding member (53), which member is connected to said tool in a defined geometrically related location to said tool when the tool is connected to the base, which member is guided by said shoe as defined by said guiding space, to control the movement of the tool to said pre-determined space.

4. The apparatus of claim 1 wherein the shoe with a defined guiding space comprises a body with a model cavity essentially defining said pre-determined space.

5. The apparatus of claim 1 wherein the tool is connected to the base (40) by a pantograph (48) which holds the guiding member(53), which pantograph is connected to the frame via a joint member (43), adjustably lockable to the base (40).

6. The apparatus of claim 5 wherein the model cavity is scaled up by a pre-determined ratio compared to the pre-determined space, and wherein said pantograph scales down the movement of said tool compared to the movement of said guiding member by the same ratio such as to guide said tool to within said pre-determined space.

7. The apparatus of any of claims 3 - 6, where the movement of the tool is steered by a joystick handle (54) connected to said guiding member (53).

8. The apparatus according to claim 1, wherein said guiding means comprise:
a) a computer system storing a digital geometric representation of said predetermined space;
b) means for registering the movement of said tool and input such movement data within said computer system after having placed the tool at a suitable starting point corresponding to a reference point of said pre-determined space;
c) means for controlling the location and/or function of said tool to within said predetermined space, by stopping the movement and/or function of said tool when the location of said tool reaches the boundary of said predetermined space.

9. The apparatus according to claim 1, wherein the guiding means for controlling the movement and/or function of said tool comprise:
a) an imaging device for obtaining data representing the tooth to be treated, wherein said data provides positional information of said tooth with reference to said base;
b) means for determining the location of said tool in relation to the tooth to be repaired;
c) a computer system which can import said data to produce a digital geometric representation of said tooth with positional coordinates in reference to said base, said system interacting with said location determining means, and interacting with said tool to control the location and/or function of said tool in relation to said tooth to a pre-determined space.

10. The apparatus according to claim 8 or 9, wherein said computer system provides the location of said tool in relation to said tooth.

11. The apparatus according to claim 10, wherein the location of the tool in relation to the tooth is visualized graphically by means of a geometric representation of the tooth.

12. The apparatus according to claim 11, wherein the computer system generates a geometric representation of said pre-deterrruned space within the tooth to be repaired, which representation may optionally be superimposed on the geometric representation of the tooth to be treated.

13. The apparatus according to claim 12, wherein the geometric representation of said pre-determined space is subtracted from said geometric representation of the tooth to be repaired to obtain a geometric representation of said tooth with a cavity to be prepared.

14. The apparatus according to claim 9, wherein said location determining means comprise displacement sensors.

15. The apparatus according to claim 8 or 9, wherein the computer system interacts with said tool to control the function of said tool such as by regulating the electrical power, airflow, laser light or water flow providing the operational function of said tool.

16. The apparatus according to claim 1, wherein said connection comprises a seat (11) which is engaged through a bearing unit (20,21) to a fixed element of the base (1), and wherein the tool is supported by said seat.

17. The apparatus according to claim 16, wherein the device is a conventional dental turbine (12) and wherein said seat (11) is shaped to firmly support said turbine.

18. The apparatus according to claim 9, wherein said imaging device comprises an image detector supported by said base.

19. The apparatus according to claim 16 and 18, wherein said detector is supported by said seat and wherein said device and detector are interchangeable in said seat.

20. The apparatus according to claim 18, wherein said image detector is a contact sensor, wherein said image data is collected by scanning the surface of said tooth with said contact sensor.

21. The apparatus according to claim 9, wherein said supported movement of the tool is remotely controlled movement.

22. The apparatus according to claim 21, wherein said movement of the tool is effected by micro-actuators, wherein said micro-actuators interact with said computer system, and wherein the apparatus further comprises a user interface to steer said movement.

23. The apparatus according to claim 21, wherein said movement is effected by micro-actuators which interact with said computer system, and wherein the computer system steers the movement based on the pre-determined space.

24. The apparatus according to claim 1, wherein said tool is connected to said base through a remote connection.

25. The apparatus according to claim 24, wherein, said remote connection comprises an electromagnetic radiation source joined to said device, which source emits one or more beams, and a spatial absorption detector comprised in said base, which detector detects the radiation emitted by said source.

26. The apparatus according to claim 1, wherein said guiding means comprise:
a) a computer system storing a digital geometric representation of said predetermined space,
b) micro-actuators interacting with said computer system to steer the movement of said tool along a path based on the pre-determined space, wherein a reference point of the path is determined by placing the tool at a selected point by the tooth to be treated.

27. The apparatus according to claim 1, wherein the base comprises:
a. a fork-shaped frame comprising two symmetric distal members with substantially parallel distal ends, the distal members connected to a proximal member which is further connected to a support member which extends out of the mouth of the subject when the frame is mounted within the mouth of said subject;
b. at least two but preferably three adjustable holders mounted on the frame, which holders are suitably shaped such as to form fixtures holding the base fixed to a jaw of teeth, when Introducing suitable molding paste in-between the holders and teeth of said jaw and allowing said paste to harden.

28. The apparatus of claim 27, wherein said proximal member of the base has a hinge for varying the angle between the distal members.

29. The apparatus of claim 27, wherein said holders of the base have a substantially curved or angled shape, allowing sufficient contact area with said molding paste, to form fixtures to said teeth when said paste has hardened.

## Patentansprüche

1. Zahnbehandlungsvorrichtung, umfassend:
a) eine Basis (40) mit Mitteln zum Befestigen der Basis im Mund eines Patienten und in einer festen Position im Verhältnis zu einem zu behandelnden Zahn,
b) Zahnbehandlungswerkzeug (51), das mit der Basis in einer Weise verbunden ist, die eine abgestützte Bewegung des Werkzeugs im Verhältnis zu der Basis erlaubt,
c) Führungsmittel (48, 53) zum Steuern der Bewegung und/oder der Funktion des Werkzeugs innerhalb eines vorbestimmten Raums.

2. Zahnbehandlungsvorrichtung nach Anspruch 1, wobei die Führungsmittel eine manuell geführte abgestützte Bewegung des Werkzeugs innerhalb des vorbestimmten Raums erlauben.

3. Zahnbehandlungsvorrichtung nach Anspruch 1, wobei die Führungsmittel Folgendes umfassen:
i. einen Aufspannkörper (46) mit einem begrenzten Führungsraum (58), wobei der Aufspannkörper mit der Basis (40) einstellbar und feststellbar in einer festen Position im Verhältnis zu der Basis verbunden ist;
ii. ein Führungselement (53), das mit dem Werkzeug in einer begrenzten, in einem geometrischen Bezug stehenden Position zu dem Werkzeug verbunden ist, wenn das Werkzeug mit der Basis verbunden ist, wobei das Element von dem Aufspannkörper geführt wird, wie es durch den Führungsraum vorgegeben ist, um die Bewegung des Werkzeugs zu dem vorbestimmten Raum zu steuern.

4. Vorrichtung nach Anspruch 1, wobei der Aufspannkörper mit dem begrenzten Führungsraum einen Körper mit einer Modellkavität umfasst, die im Wesentlichen den vorbestimmten Raum begrenzt.

5. Vorrichtung nach Anspruch 1, wobei das Werkzeug mit der Basis (40) über einen Pantograph (48) verbunden ist, der das Führungselement (53) hält, wobei der Pantograph mit dem Rahmen über ein Gelenkelement (43) verbunden ist, das verstellbar an der Basis (40) feststellbar ist.

6. Vorrichtung nach Anspruch 5, wobei die Modellaussparung im Vergleich zu dem vorbestimmten Raum in einem vorbestimmten Verhältnis vergrößert ist, und wobei der Pantograph die Bewegung des Werkzeugs im Vergleich zu der Bewegung des Führungselements mit demselben Verhältnis verkleinert, um das Werkzeug innerhalb des vorbestimmten Raums zu führen.

7. Vorrichtung nach einem der Ansprüche 3 bis 6, wobei die Bewegung des Werkzeugs durch einen Steuerhebel (54) gesteuert wird, der mit dem Führungselement (53) verbunden ist.

8. Vorrichtung nach Anspruch 1, wobei die Führungsmittel Folgendes umfassen:
a) ein Computersystem, das eine digitale geometrische Darstellung des vorbestimmten Raums speichert;
b) Mittel zum Erfassen der Bewegung des Werkzeugs und zum Eingeben solcher Bewegungsdaten in das Computersystem, nachdem das Werkzeug in eine geeignete Ausgangsposition gebracht wurde, die einem Referenzpunkt des vorbestimmten Raums entspricht;
c) Mittel zum Steuern der Position und/oder der Funktion des Werkzeugs innerhalb des vorbestimmten Raums, indem die Bewegung und/oder Funktion des Werkzeugs unterbrochen wird, wenn die Position des Werkzeugs die Grenze des vorbestimmten Raums erreicht.

9. Vorrichtung nach Anspruch 1, wobei die Führungsmittel zum Steuern der Bewegung und/oder Funktion des Werkzeugs Folgendes umfassen:
a) eine Bildgebungsvorrichtung zum Erfassen der Daten, die den zu behandelnden Zahn darstellen, wobei die Daten Positionsinformation des Zahns im Verhältnis zu der Basis bereitstellen;
b) Mittel zum Bestimmen der Position des Werkzeugs im Verhältnis zu dem zu reparierenden Zahn;
c) ein Computersystem, das die Daten importieren kann, um eine digitale geometrische Darstellung des Zahns mit Positionskoordinaten im Verhältnis zu der Basis zu erzeugen, wobei das System mit dem Mittel zum Bestimmen der Position zusammenwirkt, und mit dem Werkzeug zum Steuern der Position und/oder Funktion des Werkzeugs im Verhältnis zu dem Zahn zu einem vorbestimmten Raum hin zusammenwirkt.

10. Vorrichtung nach einem der Anspruch 8 oder 9, wobei das Computersystem die Position des Werkzeugs im Verhältnis zu dem Zahn bereitstellt.

11. Vorrichtung nach Anspruch 10, wobei die Position des Werkzeugs im Verhältnis zu dem Zahn graphisch mittels einer geometrischen Darstellung des Zahns visualisiert wird.

12. Vorrichtung nach Anspruch 11, wobei das Computersystem eine geometrische Darstellung des vorbestimmten Raums innerhalb des zu reparierenden Zahns erzeugt, wobei die Darstellung wahlweise über die geometrische Darstellung des zu behandelnden Zahns überlagert werden kann.

13. Vorrichtung nach Anspruch 12, wobei die geometrische Darstellung des vorbestimmten Raums von der geometrischen Darstellung des zu reparierenden Zahns abgezogen wird, um eine geometrische Darstellung des Zahns mit einer zu behandelnden Kavität zu erhalten.

14. Vorrichtung nach Anspruch 9, wobei die Mittel zum Bestimmen der Position Verschiebungssensoren umfassen.

15. Vorrichtung nach Anspruch 8 oder 9, wobei das Computersystem mit dem Werkzeug zusammenwirkt, um die Funktion des Werkzeugs zu steuern, wie durch Regulieren des elektrischen Stroms, des Luftstroms, des Laserlichts oder des Wasserflusses, was die Betriebsfunktion des Werkzeugs bereitstellt.

16. Die Vorrichtung nach Anspruch 1, wobei die Verbindung einen Sitz (11) umfasst, der durch eine Lagereinheit (20, 21) mit einem fest angeordneten Element der Basis (1) in Eingriff steht, und wobei das Werkzeug durch den Sitz abgestützt ist.

17. Vorrichtung nach Anspruch 16, wobei die Vorrichtung eine übliche Dentalturbine (12) ist, und wobei der Sitz (11) geformt ist, um die Turbine sicher abzustützen.

18. Vorrichtung nach Anspruch 9, wobei die Bildgebungsvorrichtung einen Bilddetektor umfasst, der von der Basis abgestützt ist.

19. Vorrichtung nach Anspruch 16 und 18, wobei der Detektor durch den Sitz abgestützt ist, und wobei die Vorrichtung und der Detektor in dem Sitz austauschbar sind.

20. Vorrichtung nach Anspruch 18, wobei der Bilddetektor ein Kontaktsensor ist, wobei die Bilddaten durch Abtasten der Fläche des Zahns mit dem Kontaktsensor gesammelt werden.

21. Vorrichtung nach Anspruch 9, wobei die abgestützte Bewegung des Werkzeugs eine entfernt gesteuerte Bewegung ist.

22. Vorrichtung nach Anspruch 21, wobei die Bewegung des Werkzeugs durch Mikrostellglieder erreicht wird, wobei die Mikrostellglieder mit dem Computersystem zusammenwirken, und wobei die Vorrichtung außerdem eine Nutzerschnittstellen aufweist, um die Bewegung zu steuern.

23. Vorrichtung nach Anspruch 21, wobei die Bewegung durch Mikrostellglieder erreicht wird, die mit dem Computersystem zusammenwirken, und wobei das Computersystem die Bewegung basierend auf dem vorbestimmten Raum steuert.

24. Vorrichtung nach Anspruch 1, wobei das Werkzeug mit der Basis über eine entfernte Verbindung verbunden ist.

25. Vorrichtung nach Anspruch 24, wobei die entfernte Verbindung eine elektromagnetische Strahlungsquelle umfasst, die an die Vorrichtung angeschlossen ist, wobei die Quelle einen oder mehrere Strahlen abgibt, sowie einen räumlichen Absorptionsdetektor, der in der Basis aufgenommen ist, wobei der Detektor die von der Quelle abgegebene Strahlung erfasst.

26. Vorrichtung nach Anspruch 1, wobei die Führungsmittel Folgendes umfassen:
a) ein Computersystem, das eine digitale geometrische Darstellung des vorbestimmten Raums speichert,
b) Mikrostellglieder, die mit dem Computersystem zusammenwirken, um die Bewegung des Werkzeugs entlang einem Weg basierend auf dem vorbestimmten Raum zu steuern, wobei ein Referenzpunkt des Wegs bestimmt wird, indem das Werkzeug an einem ausgewählten Punkt bei dem zu behandelnden Zahn angeordnet wird.

27. Vorrichtung nach Anspruch 1, wobei die Basis Folgendes umfasst:
a. einen gabelförmigen Rahmen, der zwei symmetrische distale Elemente mit im Wesentlichen parallelen distalen Enden aufweist, wobei die distalen Elemente mit einem proximalen Element verbunden sind, das außerdem mit einem Stützelement verbunden ist, das sich aus dem Mund des Patienten heraus erstreckt, wenn der Rahmen in dem Mund des Patienten angeordnet ist;
b. wenigstens zwei, aber vorzugsweise drei einstellbare Halter, die an dem Rahmen angeordnet sind, wobei die Halter in geeigneter Weise geformt sind, um Aufsätze zu bilden, um die Basis fest an einem Kinn mit Zähnen zu halten, wenn geeignete Formpaste zwischen die Halter und die Zähne des Kinns eingeführt und trocknen gelassen wird.

28. Vorrichtung nach Anspruch 27, wobei das proximale Element der Basis ein Scharnier zum Variieren des Winkels zwischen den distalen Elementen aufweist.

29. Vorrichtung nach Anspruch 27, wobei die Halter der Basis eine im Wesentlichen gekrümmte oder abgewinkelte Form aufweisen, was ausreichend Kontaktfläche mit der Formpaste zulässt, um Aufsätze auf den Zähnen zu bilden, wenn die Paste getrocknet ist.

## Revendications

1. Appareil de traitement dentaire comprenant :
a) une base (40) présentant un moyen pour attacher la base à l'intérieur de la bouche d'un patient et dans une position fixe par rapport à une dent à traiter,
b) un outil de traitement dentaire (51), connecté à ladite base d'une manière permettant le mouvement supporté de l'outil par rapport à la base,
c) des moyens de guidage (48, 53) pour contrôler le mouvement et / ou le fonctionnement dudit outil au sein d'un espace prédéterminé.

2. Appareil de traitement dentaire selon la revendication 1, dans lequel les moyens de guidage permettent un mouvement supporté dirigé manuellement dudit outil au sein dudit espace prédéterminé.

3. Appareil de traitement dentaire selon la revendication 1, dans lequel les moyens de guidage comprennent :
i. un sabot (46) avec un espace de guidage défini (58), ledit sabot étant connecté à ladite base (40) ajustable et verrouillable en place dans une position fixe sélectionnée par rapport à la base
ii. un élément de guidage (53), ledit élément étant connecté audit outil dans un emplacement défini lié géométriquement audit outil quand l'outil est connecté à la base, lequel élément est guidé par ledit sabot comme défini par ledit espace de guidage, pour contrôler le mouvement de l'outil vers ledit espace prédéterminé.

4. Appareil selon la revendication 1, dans lequel le sabot avec un espace de guidage défini comprend un corps avec une cavité modelée définissant essentiellement ledit espace prédéterminé.

5. Appareil selon la revendication 1, dans lequel l'outil est connecté à la base (40) par un pantographe (48) qui maintient l'élément de guidage (53), lequel pantographe est connecté au cadre par l'intermédiaire d'un élément de joint (43) verrouillable de manière ajustable à la base (40).

6. Appareil selon la revendication 5, dans lequel la cavité modelée est augmentée dans un rapport prédéterminé par rapport à l'espace prédéterminé, et dans lequel ledit pantographe diminue le mouvement dudit outil par rapport au mouvement dudit élément de guidage par le même rapport de manière à guider ledit outil au sein dudit espace prédéterminé.

7. Appareil selon l'une quelconque des revendications 3 à 6, dans lequel le mouvement de l'outil est dirigé par une poignée de levier de commande (54) connectée audit élément de guidage (53).

8. Appareil selon la revendication 1, dans lequel lesdits moyens de guidages comprennent :
a) un système informatique mémorisant une représentation géométrique numérique dudit espace prédéterminé ;
b) un moyen d'enregistrement du mouvement dudit outil et la saisie des données d'un tel mouvement au sein dudit système informatique après avoir placé l'outil à un point de départ approprié correspondant à un point de référence dudit espace prédéterminé ;
c) un moyen de contrôle de l'emplacement et/ou du fonctionnement dudit outil dans ledit espace prédéterminé, en arrêtant le mouvement et/ou le fonctionnement dudit outil quand l'emplacement dudit outil atteint la limite dudit espace prédéterminé.

9. Appareil selon la revendication 1, dans lequel les moyens de guidage de contrôle du mouvement et/ou du fonctionnement dudit outil comprennent :
a) un dispositif d'imagerie pour obtenir des données représentant la dent à traiter, dans lequel lesdites données fournissent des informations sur la position de ladite dent en référence à ladite base ;
b) des moyens de détermination de l'emplacement dudit outil par rapport à la dent à réparer ;
c) un système informatique qui peut importer lesdites données pour produire une représentation géométrique numérique de ladite dent avec les coordonnées de position en référence à ladite base, ledit système interagissant avec lesdits moyens de détermination de l'emplacement, et interagissant avec ledit outil de contrôle de l'emplacement et/ou du fonctionnement dudit outil par rapport à ladite dent à un emplacement prédéterminé.

10. Appareil selon la revendication 8 ou la revendication 9, dans lequel ledit système informatique fournit l'emplacement dudit outil par rapport à ladite dent.

11. Appareil selon la revendication 10, dans lequel l'emplacement de l'outil par rapport à la dent est visualisé graphiquement au moyen d'une représentation géométrique de la dent.

12. Appareil selon la revendication 11, dans lequel le système informatique créé une représentation géométrique dudit espace prédéterminé au sein de la dent à réparer, laquelle représentation peut éventuellement être superposée à la représentation géométrique de la dent à traiter.

13. Appareil selon la revendication 12, dans lequel la représentation géométrique dudit espace prédéterminé est soustraite de ladite représentation géométrique de la dent à réparer pour obtenir une représentation géométrique de ladite dent avec une cavité à préparer.

14. Appareil selon la revendication 9, dans lequel lesdits moyens de détermination de l'emplacement comprennent des capteurs de déplacement.

15. Appareil selon la revendication 8 ou la revendication 9, dans lequel le système informatique interagit avec ledit outil pour contrôler le fonctionnement dudit outil tel qu'en régulant l'alimentation électrique, le débit d'air, la lumière laser ou le débit d'eau fournissant la fonction opérationnelle dudit outil.

16. Appareil selon la revendication 1, dans lequel ladite connexion comprend un siège (11) qui entre en engagement au travers d'une unité de palier (20, 21) avec un élément fixe de la base (1), et dans lequel l'outil est supporté par ledit siège.

17. Appareil selon la revendication 16, dans lequel le dispositif est une turbine dentaire traditionnelle (12) et dans lequel ledit siège (11) est formé pour supporté fermement ladite turbine.

18. Appareil selon la revendication 9, dans lequel ledit dispositif d'imagerie comprend un détecteur d'image supporté par ladite base.

19. appareil selon la revendication 16 et la revendication 18, dans lequel ledit détecteur est supporté par ledit siège et dans lequel ledit dispositif et ledit détecteur sont interchangeables dans ledit siège.

20. Appareil selon la revendication 18, dans lequel ledit détecteur d'image est un capteur à contact, dans lequel lesdites données d'image sont recueillies par balayage de la surface de ladite dent avec ledit capteur à contact.

21. appareil selon la revendication 9, dans lequel ledit mouvement supporté de l'outil est un mouvement contrôlé à distance.

22. Appareil selon la revendication 21, dans lequel ledit mouvement de l'outil est effectué par des micro-actionneurs, dans lequel lesdits micro-actionneurs interagissent avec ledit système informatique et dans lequel l'appareil comprend en outre une interface utilisateur pour diriger ledit mouvement.

23. Appareil selon la revendication 21, dans lequel ledit mouvement est effectué par des micro-actionneurs qui interagissent avec ledit système informatique et dans lequel le système informatique dirige le mouvement en fonction de l'espace prédéterminé.

24. Appareil selon la revendication 1, dans lequel ledit outil est connecté à ladite base au travers d'une connexion à distance.

25. Appareil selon la revendication 24, dans lequel ladite connexion à distance comprend une source de radiation électromagnétique jointe audit dispositif, laquelle source émet un ou plusieurs faisceaux, et un détecteur d'absorption spatiale compris dans ladite base, lequel détecteur détecte la radiation émise par ladite source.

26. Appareil selon la revendication 1, dans lequel lesdits moyens de guidage comprennent :
a) un système informatique mémorisant une représentation géométrique numérique dudit espace prédéterminé,
b) des micro-actionneurs interagissant avec ledit système informatique pour diriger le mouvement dudit outil le long d'un chemin basé sur l'espace prédéterminé, dans lequel un point de référence du chemin est déterminé en plaçant l'outil à un point sélectionné à proximité de la dent à traiter.

27. Appareil selon la revendication 1, dans lequel la base comprend :
a. un cadre en forme de fourche comprenant deux éléments distaux symétriques avec des extrémités distales essentiellement parallèles, les éléments distaux étant connectés à un élément proximal qui est en outre connecté à un élément de support qui s'étend en dehors de la bouche du sujet quand le cadre est monté au sein de la bouche dudit sujet ;
b. au moins deux, mais de préférence trois, supports ajustables montés sur le cadre, lesquels supports étant d'une forme appropriée de manière à former des accessoires maintenant la base fixée à une mâchoire de dents lors de l'introduction d'une pâte de moulage appropriée entre les supports et les dents de ladite mâchoire et permettant à ladite pâte de durcir.

28. Appareil selon la revendication 27, dans lequel ledit élément proximal de la base présente une charnière pour varier l'angle entre les éléments distaux.

29. Appareil selon la revendication 27, dans lequel lesdits supports de la base présentent une forme essentiellement courbée ou en angle, offrant une surface de contact suffisante avec ladite pâte de moulage, pour former des accessoires sur lesdites dents quand ladite pâte a durci.
